# EUROPEAN PATENT SPECIFICATION

(11) **EP 3 372 671 B1**
(45) Date of publication and mention of the grant of the patent: **03.11.2021**
(21) Application number: 17160053.9
(22) Date of filing: 09.03.2017
(51) Int. Cl.: C12N 5/00

(54) **CULTURE MEDIUM COMPRISING OLGOPEPTIDES**
KULTURMEDIUM MIT OLIGOPEPTIDEN
MILIEU DE CULTURE COMPRENANT DES OLIGOPEPTIDES

(43) Date of publication of application: 12.09.2018
(73) Proprietor: Evonik Operations GmbH, 45128 Essen (DE)
(72) Inventor: KNAUP, Günter, 63486 Bruchköbel (DE); MERZ, Friedhelm, 55283 Nierstein (DE); KESSLER, Christian, 63741 Aschaffenburg (DE)
(74) Representative: Evonik Patent Association

(56) References cited:
- WO-A1-2012/019160
- WO-A2-01/46220
- US-A- 5 034 377
- CHALISOVA N I ET AL: "The effect of dipeptides consisting of leucine and lysine on cell proliferation in an organotypic culture of myocardium and spleen tissue from young and old rats", ADVANCES IN GERONTOLOGY, PLEIADES PUBLISHING, MOSCOW, vol. 5, no. 3, 15 October 2015 (2015-10-15), pages 180-183, XP035602531, ISSN: 2079-0570, DOI: 10.1134/S2079057015030042 [retrieved on 2015-10-15]
- F. FRANEK ET AL: "Enhancement of Monoclonal Antibody Production by Lysine-Containing Peptides", BIOTECHNOLOGY PROGRESS., vol. 19, no. 1, 7 February 2003 (2003-02-07), pages 169-174, XP055365729, US ISSN: 8756-7938, DOI: 10.1021/bp020077m
- MAQUART F X ET AL: "Stimulation of collagen synthesis in fibroblast cultures by the tripeptide-copper complex glycyl-L-histidyl-L-lysine-Cu<2+>", FEBS LETTERS, ELSEVIER, AMSTERDAM, NL, vol. 238, no. 2, 10 October 1988 (1988-10-10), pages 343-346, XP025597857, ISSN: 0014-5793, DOI: 10.1016/0014-5793(88)80509-X [retrieved on 1988-10-10]

## Description

### FIELD OF THE INVENTION

The present invention relates to biotechnological production processes. More specifically, the present invention relates to improved culture media for use in biotechnological production processes, processes employing such improved media, and to products obtained from the processes using the improved culture media.

### BACKGROUND OF THE INVENTION

Biotechnological processes are widely used for the production of biological products. These processes typically involve the cultivation of cells in a culture medium under conditions permissible to the growth and product formation by the cultivated cells. Cells useful in biotechnological production are bacterial cells, fungal cells, yeast cells, and cells of animal or plant origin.

Animal cell culture has long been used for the production of biological products, such as therapeutic proteins, polypeptides, oligopeptides or other biological molecules, such as therapeutic polysaccharides. In such cell culture processes, animal cells, normally genetically modified to produce a desired product, are cultivated in a liquid, solid or semi solid culture medium for cell proliferation and product formation. One significant advantage of cell culture is the fact that animal cells and plant cells are able to perform post-translational modifications of the primary product, such as folding and post-translational modification of a polypeptide.

In biotechnological production processes, in particular in animal cell cultures, control and optimization of the cell culture conditions is critical for cell proliferation and product formation. One decisive factor is medium composition. The concentration and quality of the final cell culture product depends heavily on the medium composition. Animal and plant cell cultures are particularly demanding in terms of the required nutrient composition and culture conditions. Required nutrients not only include basic sources for carbon, nitrogen and energy, such as sugar and ammonia, but also more complex nutrients, such as essential amino acids, growth factors and vitamins. For this reason, supplementation of animal cell culture media with complex nutrient compositions, such as serum, has been used to provide the animal cells with a broad variety of nutrients. However, regulatory and safety concerns, as well as problems with the heterogeneity of the available sources of sera has led the industry to strive towards eliminating serum and other non-defined media from industrial cell culture processes. Cell cultures grown in serum-free media, however, often show nutritional deficiencies. For this reason, much effort has been given to identify those nutritional components of complex media, as well as their optimal concentrations, which are required for satisfactory growth and protein formation in cell cultures.

The supply of cell cultures with amino acids is known to have a significant effect on growth rate and production. Glutamine is routinely used in cell culture media, since it is an important source of carbon, nitrogen and energy for the cultured cells. It was demonstrated that the amount of glutamine necessary for optimal growth of animal cell cultures is 3 to 10 times greater than the amount of other amino acids (Eagle et al., Science 130:432-37). However, glutamine as a nutrient is unstable when dissolved in water at elevated temperatures, such as heat sterilizing conditions, because pyroglutamate and ammonia are formed under heat (Roth et al. 1988, In Vitro Cellular & Developmental Biology 24(7): 696-98). For this reason, glutamate is often used in cell culture media instead of glutamine (Cell Culture Technology for Pharmaceutical and Cell-based Therapies, 52, Sadettin et al., Eds., Taylor and Francis Group, 2006). Other groups have tried to avoid the formation of unwanted substances, such as pyroglutamate and ammonia, by adding glutamine-containing dipeptides, such as alanyl-glutamine or glycyl-glutamine, to the cell culture medium (Roth et al. (1988), In Vitro Cellular & Developmental Biology 24(7): 696-98). Yet other groups have proposed acylating dipeptides in order to make them more stable under heat sterilization conditions. US 5,534,538 describes N-acyl dipeptides and their use in heat sterilized enteral or parenteral nutrition products as well as cell culture media.

Franek et al. (2002, Biotechnol. Prog. 18, 155-158; US 2004/0072341) screened various synthetic oligopeptides (including oligo-Gly, oligo-Ala) for their effect on a mouse cell line in serum-free medium. They reported that, while the single amino acid and the tested dipeptides did not significantly alter the culture performance, tri-, tetra- and penta-glycine, as well as tri- and tetra-alanine, enhanced viable cell density and cell viability.

Franek et at (2003, Biotechnol. Prog. 19, 169-174) also tested a variety of randomly selected lysine-containing peptides for their effect on Hybridoma cells using a serum-free basal medium. These were Gly-Lys, Gly-Lys-Gly, Gly-His-Lys, Lys-Lys-Lys, Gly-Phe-Gly and Gly-Gly-Lys-Ala-Ala. The authors found a suppression of cell growth and increased titers for tri-, tetra- and penta-peptides. However, conclusions are difficult to draw as the basal media also contained the peptide constituents. In addition, the authors used a concentration of 0.2 % (w/v) and did not discuss the potential implication on solubility.

WO 2011/133902 discloses cell culture media comprising dipeptides, wherein the dipeptides include amino acids having a low solubility in water, in this case tyrosine and cysteine. Cysteine does not only have a low solubility in water, but it is also unstable, due to the presence of reactive thiol group. The authors have found that by incorporation of tyrosine and cysteine in dipeptides, solubility and stability problems of the amino acids can be ameliorated. The authors also found an improved shelf life of the resulting cell culture media. In one embodiment, the remaining amino acid of the dipeptide (not cysteine or tyrosine) is asparagine.

WO 2012/019160 discloses animal cell cultures, wherein during the production phase the serum-free medium is supplemented with Tyr- and His-containing dipeptides. Positive effects of the addition of the Tyr- and His-containing dipeptides on growth and product formation are described. This also includes Tyr-Lys,

Although different dipeptides are mentioned in WO 2011/133902 and WO 2012/019160, an effect is only described for the dipeptides L-alanyl-L-tyrosine and L-alanyl-L-cystine. The solubility of these alanyl-dipeptides being 8,7 g/ltr and 15,3 g/ltr respectively, is higher as for the corresponding amino acids (cystine: 0,1 g/ltr, tyrosine: 0,4 g/ltr). However, these values are low when compared to other amino acids, whereby an application in high-concentration media like used in the feed-process, is limited. As a further disadvantage, when using alanyl-dipeptides, L-alanine is released, which is not contained in conventional cell culture media as e.g. DMEM and RPMI 1640 (Sigma) and which seems to be not required by the cells. As a matter of fact, when using L-alanyl-L-glutamine instead of L-glutamine in cell culture media, a four-fold higher concentration of alanine was found in the cell culture medium (M. Christie, J. Butler, Biotechnol. 37 (1994) 277). As cells tend to accumulate L-alanine as reported by DeBerardinis et al (2007, Proc Natl Acad Sci U S A. 104, 19345-50), there is a need for bioavailable oligopeptides without alanine. Chalisova et al. (2015, Advances in Gerontology vol. 5, no. 3, pages 180-183) discloses Lys-containing dipeptides, which are used in tissue culture, however at very low concentrations of 0.001 to 1 ng/ml.

EP 0220379 and DE3538310 disclose the use of glutamine-containing dipeptides and tripeptides in cell culture media. Glutamine is added in form of dipeptides, in order to avoid problems stemming from the instability of glutamine under elevated temperatures. The glutamine-containing dipeptides are used as a temperature insensitive glutamine source.

While cell culture media described in the above prior art provide satisfactory performance and properties for certain cell culture processes, the prior art culture media are still not optimal. There still exists a need for further improved culture media that promote better growth and product formation in biotechnological production processes.

In particular, there is a need for cell culture media comprising oligopeptides at higher concentrations, especially oligopeptides made from amino acids contained in standard media, such as e.g. DMEM and RPMI-1640. Further, such oligopeptides should be easy to prepare without the need of using specific protecting groups.

### SUMMARY OF THE INVENTION

The above shortcomings of known culture media are addressed by the present invention. The invention is defined by the terms of the appended independent claims. Preferred embodiments of the invention are defined by the dependent claims. The invention thus relates to a culture medium, preferably a cell culture medium, comprising at least one dipeptide of 2 amino acid units in length, said amino acid units being natural amino acids, and at least one amino acid unit being lysine (Lys), wherein the oligopeptide does not comprise glycine (Gly) and glutamic acid (Glu), wherein the dipeptide is Lys-Xxx, wherein Xxx is selected from Cys, Cyss, Tyr, Val, Leu, and Ile, and wherein said oligopeptide is present in said culture medium at a concentration of from 0.1 to 10 mM.

The invention further relates to the use of a culture medium of the invention for culturing cells, preferably plant cells, animal cells or mammalian cells.

Another aspect of the invention relates to a method of manufacturing a cell culture product comprising the steps of (i) providing a cell capable of producing said cell culture product; (ii) contacting said cell with a culture medium according to the invention; and (iii) obtaining said cell culture product from said culture medium or from said cell.

Preferred embodiments of the invention are described in further detail in the following detailed description of the invention.

### BRIEF DESCRIPTION OF THE FIGURES

Figure 1 depicts the maximum viable cell density with TC-42 media obtained with oligopeptides according to the invention.
Figure 2 depicts the maximum viable cell density with TC-42 media obtained with oligopeptides according to the invention.
Figure 3 depicts the maximum viable cell density with TC-42 media obtained with oligopeptides according to the invention.

### DETAILED DESCRIPTION OF THE INVENTION

A "culture medium", according to the invention, shall be understood as being a liquid or solid medium containing nutrients, the medium being suitable for nourishing and supporting life and/or product formation of cells in the culture. The cultured cells, according to the invention, may be bacterial cells, yeast cells, fungal cells, animal cells, such as mammalian cells or insect cells, and/or plant cells, e.g., algae. Typically, a culture medium provides essential and non-essential amino acids, vitamins, at least one energy source, lipids, and trace elements, all required by the cell for sustaining life, growth and/or product formation. The culture medium may also contain components that enhance growth and/or survival above the minimal rate, including hormones and growth factors. The culture medium has preferably a pH and a salt concentration which supports life, growth and/or product formation of the cells. A culture medium, according to the invention, preferably comprises all nutrients necessary to sustain life and proliferation of the cell culture. Preferred culture media are defined media.

A "defined medium", according to the invention is a medium that contain no cell extracts, cell hydrolysates, or protein hydrolysates. Preferred defined media comprise no components of unknown composition. As is commonly understood by the person skilled in the art, defined media are usually free of animal-derived components. Preferably, all components of a defined medium have a known chemical structure. Preferred defined media are serum-free media. Other preferred defined media are synthetic media. Culture media other than defined culture media are referred to as "complex" culture media.

A "cell culture medium" shall be understood as being a culture medium suitable for sustaining life, proliferation and/or product formation of animal cells and/or plant cells. In certain embodiments, one can distinguish between a basal medium and a feed medium.

A "basal medium" shall be understood as being a solution or substance containing nutrients in which a culture of cells is initiated. A "feed medium" shall be understood as being a solution or substance with which the cells are fed after the start of the cultivation process. In certain embodiments, a feed medium contains one or more components not present in a basal medium. The feed medium can also lack one or more components present in a basal medium. Preferably, the concentration of nutrients in the feed medium exceeds the concentration in the basal medium to avoid a loss of productivity by dilution.

A "nutrient", according to the present invention, is a chemical compound or substance which is needed by cells to live and grow. The nutrient is preferably taken up by the cell from the environment. Nutrients can be "organic nutrients" and "inorganic nutrients". Organic nutrients include carbohydrates, fats, proteins (or their building blocks, e.g., amino acids), and vitamins. Inorganic nutrients are inorganic compounds such as, e.g., dietary minerals and trace elements. "Essential nutrients" are nutrients which the cell cannot synthesize itself, and which must thus be provided to the cell by the culture medium.

A "cell culture product", according to the invention, shall be understood as being any useful biological compound produced by cells in cell culture. Preferred cell culture products of the invention are therapeutic proteins, diagnostic proteins, therapeutic polysaccharides, such as heparin, antibodies, e.g., monoclonal antibodies, growth factors, interleukin, peptide hormones, enzymes, and viruses. In other embodiments, including but not limited to the cultivation of stem cells for therapeutic use, the cell itself shall be understood as the cell culture product.

An "amino acid", in the context of the present invention, shall be understood as being a molecule comprising an amino functional group (-NH₂) and a carboxylic acid functional group (-COOH), along with a side-chain specific to the respective amino acid. In the context of the present invention, both alpha- and beta-amino acids are included. Preferred amino acids of the invention are alpha-amino acids, in particular the 20 "natural amino" acids including cystine as follows (table 1):
- Alanine: (Ala/A)
- Arginine: (Arg/R)
- Asparagine: (Asn / N)
- Aspartic acid: (Asp / D)
- Cysteine: (Cys / C)
- Cystine: (Cyss/C2)Glutamic acid (Glu / E)
- Glutamine: (Gln / Q)
- Glycine: (Gly / G)
- Histidine: (His / H)
- Isoleucine: (Ile / l)
- Leucine: (Leu / L)
- Lysine: (Lys / K)
- Methionine: (Met / M)
- Phenylalanine: (Phe / F)
- Proline: (Pro / P)
- Serine: (Ser / S)
- Threonine: (Thr / T)
- Tryptophan: (Trp / W)
- Tyrosine: (Tyr / Y)
- Valine: (Val/V)

In the context of this inventions, Cys-peptides forming a disulfide bond, thus containing cysteine, shall be described by X₂-Cyss.

In the context of the present invention, the expression "natural amino acids" shall be understood to include both the L-form and the D-form of the above listed 20 amino acids. The L-form, however, is preferred. In one embodiment, the term "amino acid" also includes analogues or derivatives of those amino acids.

A "free amino acid", according to the invention, is understood as being an amino acid having its amino and its (alpha-) carboxylic functional group in free form, i.e., not covalently bound to other molecules, e.g., an amino acid not forming a peptide bond. Free amino acids may also be present as salts or in hydrate form. When referring to an amino acid as a part of, or in, an oligopeptide, this shall be understood as referring to that part of the respective oligopeptide structure derived from the respective amino acid, according to the known mechanisms of biochemistry and peptide biosynthesis.

A "growth factor", according to the invention, shall be understood as being any naturally occurring substance capable of stimulating cellular growth, proliferation and cellular differentiation. Preferred growth factors are in form of protein or steroid hormone. According to one embodiment of the invention, the expression "growth factor" shall be interpreted as relating to a growth factor selected from the list consisting of fibroblast growth factor (FGF), including acidic FGF and basic FGF, insulin, insulin-like growth factor (IGF), epithelial growth factor (EGF), nerve growth factor (NGF), platelet-derived growth factor (PDGF), and transforming growth factor (TGF), including TGFalpha and TGFbeta, cytokine, such as interleukins 1, 2, 6, granulocyte stimulating factor, and leukocyte inhibitory factor (LIF).

An "oligopeptide", as described herein shall be understood as being a peptide compound consisting of 2 to 20 amino acids. More preferred oligopeptides are oligopeptides consisting of 2-10 amino acids, 2-6 amino acids, 2-5 amino acids, 2-4 amino acids, or 2-3 amino acids. Oligopeptides according to the invention are dipeptides.

A "peptide" shall be understood as being a molecule comprising at least two amino acids covalently coupled to each other by a peptide bond (R¹-CO-NH-R²).

The expression "Xxx", when used herein in connection with an amino acid, shall be understood as referring to any natural amino acid as listed hereinabove.
The expression "N-acylated", with reference to a chemical compound, such as an amino acid, shall be understood as meaning that the N-acylated compound is modified by the addition of an acyl group to a nitrogen functional group of said compound. Preferably, the acyl group is added to the alpha-amino group of the amino acid.

A "sterile form" of a nutrient composition, culture medium, cell culture medium, or the like, shall be understood as defining the absence of any living matter in said composition, culture medium, cell culture medium or the like.

A "solid culture medium", in the context of the present invention, shall be understood as being any non-liquid or non-gaseous culture medium. Preferred solid culture media of the invention are gel-like culture media, such as agar-agar, carrageen or gelatine.

"Passaging of cells" (also known as subculture or splitting of cells), in the context of the present invention, is understood as meaning the transferring a small number of cells into a new culture vessel. Cells can be cultured for a longer time if they are split regularly, as it avoids the senescence associated with prolonged high cell density. Suspension cultures are easily passaged with a small amount of culture containing a few cells diluted in a larger volume of fresh media.

The present invention generally relates to a culture medium, preferably a cell culture medium, said culture medium comprising at least one dipeptide of 2 amino acids in length, said amino acids being natural amino acids, and at least one amino acid unit being lysine (Lys), wherein the oligopeptide does not comprise glycine (Gly) and glutamic acid (Glu), wherein the dipeptide is Lys-Xxx, wherein Xxx is selected from Cys, Cyss, Tyr, Val, Leu, and Ile, and wherein said oligopeptide is present in said culture medium at a concentration of from 0.1 to 10 mM. The use of lysine avoids the accumulation of wasteful amino acids such as alanine which is a key constituent of most commercially available peptides for cell culture applications.

The dipeptide Tyr-Lys is preferably excluded.

According to the present invention the or N-terminal amino acid is a lysine unit. It shall be noted that the C- and N-terminal sequence can have an influence on the cell culture as evidenced by the different CAS number.

Surprisingly, it was found that the lysine peptides have a higher solubility than what should be expected based on published data (calculated using Scifinder and the Advanced Chemistry Development (ACD/Labs) Software V11.02 (© 1994-2016 ACD/Labs). For Bis-L-Lysyl-L-cystin hydrochloride, a solubility of > 50% (w/w) was found, which exceeds the calculated solubility of 4.2 % (w/w) considerably.

In table 1 the solubilities of different amino acids and dipeptides is summarized. It was also found that the preferred embodiments of lysine peptides furthermore allow cells to grow, in contrast to other lysine-peptides which did not lead to cell growth. This is surprising as A. Nagamatsu und T. Hayashida (Chemical & Pharmaceutical Bulletin 22, 2680 (1974)) have analyzed the hydrolisis of Lys-Leu, Lys-Tyr and Lys-Lys with plasmin and trypsin and found that none of these peptides were cleaved. By combining high solubility with bioavailability, lys-peptides offer new ways to solve the problem of preparing cell culture media and feeds with high amino acid concentrations.

**Table 1: Solubilities of aminoacids and dipeptides (g / 1000 g solution)**

| **Amino acid** | **Xxx¹)** | **Gly-Xxx⁵)** | **Ala-Xxx**⁵) | **Lys-Xxx calc**³) | **Lys-Xxx found** |
|---|---|---|---|---|---|
| L-Cystine | 0,1 | 50⁴) | 8,7²) | 42 | > 500⁶) |
| L-Tyrosine | 0,4 | 33 | 15,3 | 21 | > 330⁷) |
| L-Valine | 81,3 | 370 | 102 | 287 | > 400⁶) |
| L-Leucine | 23,7 | 70 | 248 | 200 | > 330⁷) |
| L-Isoleucine | 39,6 | 210 | 86 | 210 | > 500⁷) |

| | | | | | |
|---|---|---|---|---|---|
| 1) CRC Handbook of Chemistry and Physics, 58th Ed., 1977, page C-769 2) WO 2011/133902, Life Techn., page 40 3) SciFinder, Calculated using Advanced Chemistry Development (ACD/Labs) Software V11.02 (© 1994-2016 ACD/Labs) 4) MSDS Bachem AG 5) Product Brochure Peptides, Degussa AD, GB Industrie- und Feinchemikalien, 1988 6) as hydrochloride 7) as acetate | | | | | |

According to the present invention the oligopeptide is a dipeptide.

The present inventors found that culture media of this kind have superior properties over prior art cell culture media.

The dipeptides Lys-Xxx may be synthesized by simply coupling a doubly N-protected lysine with one or more amino acids. This is significantly easier to do than the synthesis of the reverse sequence Xxx-Lys, for the synthesis of which not only an N-protected amino acid Xxx is necessary but also a selectively ε-protected lysine. Common derivatives of lysine for the synthesis of the dipeptides Lys-Xxx are for instance bis-N,N-carboxybenzyl-L-lysine (Z-Lys(Z)-OH) and bis-N,N-tert.-butyloxycarbonyl-L-lysine(Boc-Lys(Boc)-OH).

In the pH range, in which the cell culture mediums are used (usually pH 7.2 - 7.4), lysyl dipeptides are in the form of their salts resulting from the protonation of one of the amino groups. For the formation of salts all acids may be used that are compatible with the cell culture. The most common salts are chloride, sulphate, nitrate, phosphate, and carbonate. However, organic acids like for instance formic acid, acetic acid, lactic acid, glutamic acid, or aspartic acid may be used as well.

The lysyl dipeptides dissolve in water significantly more readily than the corresponding alanyl dipeptides.

According to the present invention the oligopeptide is Lys-Xxx, wherein Xxx is any natural amino acid as defined hereinabove, excluding Gly.

In one embodiment, an amino acid with a low solubility, including but not limited to Tyr, Cys, Leu, Ile, Val, is replaced fully or partially by a Lys-Xxx peptide. At the same time, the amount of lysine (either in salt or salt-free form) is reduced by the same amount. Because of the high demand of the cell for lysine, the replacement of the amino acid with a low solubility by the Lys-Xxx Lys peptide can be realized without exceeding the lysine amount in the original formulation.

In preferred embodiments, the oligopeptide is not N-acylated. N-acylation is known to improve heat stability of certain oligopeptides; however, it has been found that N-acylated oligopeptides may also lead to inferior viable cell density and viability.

In one embodiment, the culture medium further comprises at least one carbohydrate, at least one free amino acid, at least one inorganic salt, a buffering agent and/or at least one vitamin. In a particularly preferred embodiment, the culture medium comprises all of at least one carbohydrate, at least one free amino acid, at least one inorganic salt, a buffering agent and at least one vitamin.

In one embodiment of the invention, the culture medium does not contain a growth factor. In accordance with this embodiment, the oligopeptide of the invention may be used instead of a growth factor for promoting growth and/or proliferation of the cells in culture. In another embodiment of the invention, the culture medium does not contain any lipids.

According to another embodiment of the invention, the culture medium is in liquid form, in form of a gel, a powder, a granulate, a pellet or in form of a tablet.
In preferred embodiments, the culture medium of the invention is a defined medium, or a serum-free medium. For example, oligopeptides of the invention may be supplemented to the CHOMACS CD medium of Miltenyi Biotech (Bergisch Gladbach, Germany), to the PowerCHO-2 CD medium available from LONZA (Basel, Switzerland), the Acti-CHO P medium of PAA (PAA Laboratories, Pasching, Austria), the Ex-Cell CD CHO medium available from SAFC, the SFM4CHO medium and the CDM4CHO medium of ThermoFisher (Waltham, USA). The oligopeptides of the invention may also be supplemented to DMEM medium (Life Technologies Corp., Carlsbad, USA). The invention, however, is not limited to supplementation of the above media.

In other preferred embodiments, the culture medium is a liquid medium in 2-fold, 3-fold, 3.33-fold, 4-fold, 5-fold or 10-fold concentrated form (volume/volume), relative to the concentration of said medium in use. This allows preparation of a "ready-to-use" culture medium by simple dilution of the concentrated medium with the respective volume of sterile water. Such concentrated forms of the medium of the invention may also be used by addition of the same to a culture, e.g., in a fed-batch cultivation process.

Culture media of the present invention preferably contain all nutrients required for sustained growth and product formation. Recipes for preparing culture media, in particular cell culture media, are well known to the person skilled in the art (see, e.g., Cell Culture Technology for Pharmaceutical and Cell-Based Therapies, Öztürk and Wei-Shou Hu eds., Taylor and Francis Group 2006). Various culture media are commercially available from various sources.

The culture media of the invention preferably include a carbohydrate source. The main carbohydrate used in cell culture media is glucose, routinely supplemented at 5 to 25 nM. In addition, any hexose, such as galactose, fructose, or mannose or a combination may be used.

The culture medium typically also includes at least the essential amino acids (i.e., His, Ile, Leu, Lys, Met, Phe, Thr, Try, Val) as well as certain non-essential amino acids. A non-essential amino acid is typically included in the cell culture medium if the cell line is not capable of synthesizing the amino acid or if the cell line cannot produce sufficient quantities of the amino acid to support maximal growth. In addition, mammalian cells can also use glutamine as a major energy source. Glutamine is often included at higher concentrations than other amino acids (2-8 mM). However, as noted above, glutamine can spontaneously break down to form ammonia and certain cell lines produce ammonia faster, which is toxic.

The culture media of the invention preferably comprise salts. Salts are added to cell culture medium to maintain isotonic conditions and prevent osmotic imbalances. The osmolality of a culture medium of the invention is about 300 mOsm/kg, although many cell lines can tolerate an approximately 10 percent variation of this value or higher. The osmolality of some insect cell cultures tend to be higher than 300 mOsm/kg, and this may be 0.5 percent, 1 percent, 2 to 5 percent, 5- 10 percent, 10-15 percent, 15- 20 percent, 20-25 percent, 25-30 percent higher than 300 mOsm/kg. The most commonly used salts in cell culture medium include Na⁺, K⁺, Mg²⁺, Ca²⁺, Cl , SO₄²⁻, PO₄³⁻, and HCO₃⁻ (e.g., CaCl₂, KCI, NaCl, NaHCO₃, Na₂HPO₄).

Other inorganic elements may be present in the culture medium. They include Mn, Cu, Zn, Mo, Va, Se, Fe, Ca, Mg, Si, and Ni. Many of these elements are involved in enzymatic activity. They may be provided in the form of salts such as CaCl₂, Fe(NO₃)₃, MgCl₂, MgSO₄, MnCl₂, NaCl, NaHCO₃, Na₂HPO₄, and ions of the trace elements, such as, selenium, vanadium and zinc. These inorganic salts and trace elements may be obtained commercially, for example from Sigma (Saint Louis, Missouri).

The culture media of the invention preferably comprise vitamins. Vitamins are typically used by cells as cofactors. The vitamin requirements of each cell line vary greatly, although generally extra vitamins are needed if the cell culture medium contains little or no serum or if the cells are grown at high density. Exemplary vitamins preferably present in culture media of the invention include biotin, choline chloride, folic acid, i-inositol, nicotinamide, D-Ca⁺⁺⁻pantothenate, pyridoxal, riboflavin, thiamine, pyridoxine, niacinamide, A, B₆, B₁₂, C, D₃, E, K, and p-aminobenzoic acid (PABA).

Culture media of the invention may also comprise serum. Serum is the supernatant of clotted blood. Serum components include attachment factors, micronutrients (e.g., trace elements), growth factors (e.g., hormones, proteases), and protective elements (e.g., antitoxins, antioxidants, antiproteases). Serum is available from a variety of animal sources including human, bovine or equine serum. When included in cell culture medium according to the invention, serum is typically added at a concentration of 5-10 %(vol.). Preferred cell culture media are serum-free.

To promote cell growth in the absence or serum or in serum reduced media, one or more of the following polypeptides can be added to a cell culture medium of the invention: for example, fibroblast growth factor (FGF), including acidic FGF and basic FGF, insulin, insulin-like growth factor (IGF), epithelial growth factor (EGF), nerve growth factor (NGF), platelet-derived growth factor (PDGF), and transforming growth factor (TGF), including TGFalpha and TGFbeta, any cytokine, such as interleukins 1, 2, 6, granulocyte stimulating factor, leukocyte inhibitory factor (LIF), etc.

However, the culture medium of the invention may also include none of the above listed growth factors. According to this aspect of the invention, the oligopeptide of the invention is used instead of any growth factor for promoting proliferation of the cells. In other words, according to this aspect of the invention, the presence of the oligopeptide of the invention makes the presence of a growth factor dispensable.

In other embodiments, the cell culture medium does not comprise polypeptides (i.e., peptides with more than 20 amino acids).

One or more lipids can also be added to a cell culture medium of the invention, such as linoleic acid, linolenic acid, arachidonic acid, palmitoleic acid, oleic acid, polyenoic acid, and/or fatty acids of 12, 14, 16, 18, 20, or 24 carbon atoms, each carbon atom branched or unbranched), phospholipids, lecithin (phophatidylcholine), and cholesterol. One or more of these lipids can be included as supplements in serum-free media. Phosphatidic acid and lysophosphatidic acid stimulate the growth of certain anchorage-dependent cells, such as MDCK, mouse epithelial, and other kidney cell lines, while phosphatidylcholine, phosphatidylethanolamine, and phosphatidylinositol stimulate the growth of human fibroblasts in serum-free media. Ethanolamine and cholesterol have also been shown to promote the growth of certain cell lines. In certain embodiment, the cell culture medium does not contain a lipid.

One or more carrier proteins, such as bovine serum albumin (BSA) or transferrin, can also be added to the cell culture medium. Carrier proteins can help in the transport of certain nutrients or trace elements. BSA is typically used as a carrier of lipids, such as linoleic and oleic acids, which are insoluble in aqueous solution. In addition, BSA can also serve as a carrier for certain metals, such as Fe, Cu, and Ni. In protein-free formulations, non-animal derived substitutes for BSA, such as cyclodextrin, can be used as lipid carriers.

One or more attachment proteins, such as fibronectin, laminin, and pronectin, can also be added to a cell culture medium to help promote the attachment of anchorage-dependent cells to a substrate.

The cell culture medium can optionally include one or more buffering agents. Suitable buffering agents include, but are not limited to, N-[2-hydroxyethyl]-piperazine- N'-[2-ethanesulfonic acid] (HEPES), MOPS, MES, phosphate, bicarbonate and other buffering agents suitable for use in cell culture applications. A suitable buffering agent is one that provides buffering capacity without substantial cytotoxicity to the cells cultured. The selection of suitable buffering agents is within the ambit of ordinary skill in the art of cell culture.

Polyanionic or polycationic compounds may be added to the culture medium to prevent the cells from clumping and to promote growth of the cells in suspension.

In a preferred embodiment, the culture medium is in liquid form. The culture medium, however, can also be a solid medium, such as a gel-like medium, e.g. an agar-agar-, carrageen- or gelatine-containing medium.

Preferably, the culture medium is in sterile form.

In preferred embodiments of the invention, the Lys-containing oligopeptide is present in said culture medium in a concentration of from 0.01 to 20 g/l, or 0.1 to 10 g/l, or 0.5 to 5 g/l. In other preferred embodiments of the invention, the Lys-containing oligopeptide is present in a concentration of above 0.01, 0.02, 0.04, 0.08, 0.2, 0.4, or 1 mM. Also preferred are culture media in which the Lys-containing oligopeptide is present in a concentration of less than 50, 20, 10, 5, or 2 mM. Preferably, the Lys-containing oligopeptide is present in the medium at a concentration of from 0.01 mM to 40 mM, or 0.1 mM to 20 mM, or 0.1 mM to 10 mM, or 0.5 mM to 10 mM, or 1 mM to 10 mM, or 1 mM to 8 mM, or 1 mM to 6 mM.

The above concentrations are given as concentrations in the non-concentrated medium, i.e., the concentration as present in the actual culture. Concentrated media may include X-fold higher concentrations.

In certain embodiments the cell culture medium of the invention comprises both Lys-containing oligopeptides and free Lys. In one preferred embodiment, the Lys-containing oligopeptide is added to a commercially available culture medium, e.g., to a defined cell culture medium or to a complex cell culture medium.

The culture medium of the present invention can be in concentrated form. It may be, e.g., in 2-fold, 3-fold, 3.33-fold, 4-fold, 5-fold, 10-fold, 20-fold, 50-fold or 100-fold concentrated form (relative to a concentration that supports growth and product formation of the cells). Such concentrated culture media are helpful for preparing the culture medium for use by dilution of the concentrated culture medium with an aqueous solvent, such as water. Such concentrated culture media may be used in batch culture, but are also advantageously used in fed-batch or continuous cultures, in which a concentrated nutrient composition is added to an ongoing cultivation of cells, e.g., to replenish nutrients consumed by the cells during culture.

In other embodiments of the invention, the culture medium is in dry form, e.g., in form of a dry powder, or in form of granules, or in form of pellets, or in form of tablets.

The present invention also relates to the use of a culture medium of the invention for culturing cells. Another aspect of the invention relates to the use of a culture medium of the invention for producing a cell culture product.

A preferred embodiment of the invention relates to the use of a culture medium according to the invention for culturing animal cells or plant cells, most preferred mammalian cells. In specific embodiments the cells to be cultured are CHO cells, COS cells, VERO cells, BHK cells, HEK cells, HELA cells, AE-1 cells, insect cells, fibroblast cells, muscle cells, nerve cells, stem cells, skin cells, endothelial cells and hybridoma cells. Preferred cells of the invention are CHO cells and hybridoma cells. Most preferred cells of the invention are CHO cells. Particularly preferred CHO cells of the invention are CHO DG44 and CHO DP12 cells.

Also included in the scope of the present invention is a method of culturing cells, said method comprising contacting said cells with a cell culture medium according to the invention. In one embodiment of the invention, the method of culturing cells comprises contacting the cell with a basal culture medium under conditions supporting the cultivation of the cell and supplementing the basal cell culture medium with a concentrated medium according to the present invention. In preferred embodiments, the basal culture medium is supplemented with the concentrated feed or medium on more than one day.

Another aspect of the invention relates to a method of producing a culture medium according to the invention, wherein said culture medium comprises at least one oligopeptide according to the invention. Methods of producing a culture medium according to the invention comprise at least one step of adding an oligopeptide of the invention to the culture medium. Likewise, an aspect of the invention relates to the use of a Lys-containing oligopeptide of the invention for producing a cell culture medium.

Another aspect of the invention relates to a method of modifying a culture medium, wherein said modifying of said culture medium comprises addition of at least one oligopeptide of the invention to said culture medium.

Another aspect of the invention relates to a method of producing a liquid culture medium, said method comprising providing solid medium according to the invention, e.g., in form of a dry powder, or in form of granules, or in form of pellets, or in form of tablets; and dissolving said solid culture medium in an aqueous medium, such as water.

Another aspect of the invention relates to the use of an oligopeptide according to the invention in a culture medium for culturing cells. Another aspect of the invention relates to the use of an oligopeptide according to the invention for cell culture.

The invention also relates to methods of manufacturing a cell culture product comprising the steps of (i) providing a cell capable of producing said cell culture product; (ii) contacting said cell with a culture medium of the invention; and (iii) obtaining said cell culture product from said culture medium or from said cell. Likewise, the present invention relates to the use of an oligopeptide according to the invention for manufacturing a cell culture product.

In preferred methods, the cell culture product is a therapeutic protein, a diagnostic protein, a polysaccharide, such as heparin, an antibody, a monoclonal antibody, a growth factor, an interleukin, virus, virus-like particle or an enzyme

Cultivation of cells, according to the invention can be performed in batch culture, in fed-batch culture or in continuous culture.

### EXAMPLES

### General Procedure

The influence of the L-lysine dipeptides on the growth of antibody producing Chinese Hamster Ovary (CHO) cells (Subclone DG44; Life Technologies Cooperation, Carslbad, USA) in comparison to media containing either the corresponding free amino acids or the corresponding L-alanine dipeptides was investigated. For this purpose, the cells were cultivated in a TC-42 media produced by Xell AG, Bielefeld, Germany in which the amino acids provided within the experiments by dipeptides were lacking. The concentrations of these amino acids were adjusted in such a way that, taking into account a complete hydrolysis of the dipeptides into the amino acids, the concentration of all amino acids were the same in all media tested within this experiment.

Starting from a preparatory culture an initial viable cell density (VCD) of 0.3-10⁶ cells/ml was adjusted in shaking flasks by adding the test media. After 2-4 days a part of the cell culture was transferred into a new shaking flask and an initial viable cell density (VCD) of 0.3-10⁶ cells/ml was adjusted by adding new test media. This was done two times and then the cell growth was followed by measuring the viable cell density (VCD) for up to 24 days.

### Experiment 1: Comparison of the cell growths obtained with TC-42 media in which L-cysteine/L-cystine is replaced by bis-L-alanyl-L-cystine, bis-L-prolyl-L-cystine or bis-L-lysyl-L-cystine and L-leucine is replaced by L-lysysl-L-leucine

For this experiment a TC-42 media produced by Xell AG, Bielefeld, Germany in which L-alanine, L-lysine, L-proline, L-cysteine, L-cystine and L-leucine were deliberately left out of the initial formulation. These amino acids and the dipeptides bis-L-alanyl-L-cystine, bis-L-prolyl-L-cystine, bis-L-lysyl-L-cystine tetrahydrochloride and L-lysyl-L-leucine, respectively, were then added to the test media in the concentration listed in table 2.

**Table 2: Concentration of amino acids and dipeptides used in the test media of experiment 1.**

| | | **Reference Media** | | **Ala₂-Cyss** | | **Pro₂-Cyss** | | **Lys₂-Cyss** | | **Lys-Leu** | |
|---|---|---|---|---|---|---|---|---|---|---|---|
| | **MW** | **[mM]** | **[mg/l]** | **[mM]** | **[mg/l]** | **[mM]** | **[mg/l]** | **[mM]** | **[mg/l]** | **[mM]** | **[mg/l]** |
| L-Alanine | 89,09 | 1,29 | 114,8 | | | 1,29 | 114,8 | 1,29 | 114,8 | 1,29 | 114,8 |
| L-Cysteine | 121,16 | 2,33 | 282,0 | | | | | | | 2,33 | 282,0 |
| L-Cystine | 240,3 | 0,12 | 30,0 | | | | | | | 0,12 | 30,0 |
| L-Leucine | 131,18 | 3,34 | 438,0 | 3,34 | 438,0 | 3,34 | 438,0 | 3,34 | 438,0 | 0,43 | 57,0 |
| L-Lysine x HCl | 182,65 | 2,90 | 530,5 | 2,90 | 530,5 | 2,90 | 530,5 | 1,62 | 295,1 | | |
| L-Proline | 115,13 | 1,29 | 148,4 | 1,29 | 148,4 | | | 1,29 | 148,4 | 1,29 | 148,4 |
| Bis-L-alanyl-L-cystine | 382,46 | | | 1,29 | 492,8 | | | | | | |
| Bis-L-prolyl-L-cystine | 434,53 | | | | | 1,29 | 559,9 | | | | |
| Bis-L-lysyl-L-cystine x 4HCl | 642,49 | | | | | | | 1,29 | 827,9 | | |
| L-Lysyl-L-leucine acetat | 319,4 | | | | | | | | | 2,90 | 927,7 |

The viable cell densities (VCD) obtained with the media from table 2 at different times are listed in table 3.

**Table 3: Viable cell densities (VCD) and maximum cell densities (max VCD) obtained with the media listed in table 2.**

| **Time** | **Reference** | **Ala₂-Cyss** | **Lys₂-Cyss** | **Pro₂-Cyss** | **Lys-Leu** |
|---|---|---|---|---|---|
| 0,0 | 0,32 | 0,26 | 0,31 | 0,31 | 0,33 |
| 0,7 | 0,46 | 0,42 | 0,39 | 0,37 | 0,39 |
| 2,0 | 1,05 | 0,47 | 0,54 | 0,32 | 0,59 |
| 2,7 | 1,32 | 0,67 | 0,83 | 0,21 | 0,81 |
| 3,6 | 1,68 | 0,84 | 1,02 | 0,18 | 0,92 |
| 3,7 | 0,37 | 0,36 | 0,39 | 0,30 | 0,34 |
| 4,7 | 0,71 | 0,71 | 0,62 | 0,24 | 0,56 |
| 5,8 | 1,48 | 1,52 | 1,18 | 0,14 | 0,94 |
| 6,7 | 2,20 | 2,34 | 1,98 | 0,08 | 1,40 |
| 6,8 | 0,34 | 0,32 | 0,30 | 0,32 | 0,30 |
| 7,8 | 0,51 | 0,48 | 0,43 | 0,43 | 0,45 |
| 8,7 | 1,03 | 1,06 | 0,98 | 0,41 | 0,79 |
| 9,7 | 2,05 | 1,79 | 1,82 | 0,34 | 1,24 |
| 10,6 | 3,21 | 3,19 | 2,88 | 0,27 | 1,81 |
| 11,6 | 4,79 | 4,89 | 4,75 | 0,79 | 2,38 |
| 12,8 | 5,93 | 7,65 | 6,68 | | 3,12 |
| 13,6 | 7,79 | 10,01 | 9,20 | | 3,66 |
| 15,0 | 9,98 | 11,62 | 11,93 | | 4,52 |
| 15,9 | 12,67 | 15,06 | 13,66 | | 5,47 |
| 16,7 | 13,80 | 14,38 | 13,41 | | 6,10 |
| 17,8 | 15,04 | 14,05 | 14,16 | | 6,03 |
| 18,7 | 15,57 | 14,28 | 14,15 | | 7,23 |
| 19,9 | 16,80 | | | | 10,51 |
| 20,7 | 18,54 | | | | 12,43 |
| 21,8 | 18,30 | | | | 12,15 |
| 22,8 | 18,46 | | | | 11,82 |
| **max. VCD** | 18,54 | 15,06 | 14,16 | 0,79 | 12,43 |

### Experiment 2: Comparison of the cell growths obtained with TC-42 media in which L-tyrosine is replaced by L-alanyl-L-tyrosine or L-lysyl-L-tyrosine

For this experiment a TC-42 media produced by Xell AG, Bielefeld, Germany in which L-alanine, L-lysine and L-tyrosine were lacking was used. These amino acids and the dipeptides L-alanyl-L-tyrosine dihydrate and L-lysyl-L-tyrosine acetate, respectively, were then added to the test media in the concentration listed in table 4.

**Table 4: Concentration of amino acids and dipeptides used in the test media of experiment 2.**

| | | **Reference Media** | | **Ala-Tyr** | | **Lys-Tyr** | |
|---|---|---|---|---|---|---|---|
| | **MW** | **[mM]** | **[mg/l]** | **[mM]** | **[mg/l]** | **[mM]** | **[mg/l]** |
| L-Alanine | 89,09 | 1,22 | 108,7 | | | 1,22 | 108,7 |
| L-Lysine x HCI | 182,65 | 2,90 | 530,5 | 2,90 | 530,5 | 1,69 | 307,7 |
| L-Tyrosine | 115,13 | 1,22 | 221,0 | | | | |
| L-Alanyl-L-tyrosine x 2 H₂O | 288,30 | | | 1,22 | 351,6 | | |
| L-Lysyl-L-tyrosine acetate | 309,37 | | | | | 1,22 | 377,34 |

The viable cell densities (VCD) obtained with the media from table 4 at different times are listed in table 5.

**Table 5: Viable cell densities (VCD) and maximum cell densities (max VCD) obtained with the media listed in table 4.**

| **Time [d]** | **Reference** | **Ala-Tyr** | **Lys-Tyr** |
|---|---|---|---|
| 0,0 | 0,43 | 0,38 | 0,42 |
| 0,7 | 0,63 | 0,51 | 0,53 |
| 1,7 | 1,33 | 1,22 | 0,82 |
| 2,7 | 2,03 | 2,22 | 1,68 |
| 2,7 | 0,39 | 0,35 | 0,39 |
| 4,0 | 0,97 | 0,77 | 0,87 |
| 4,9 | 1,95 | 1,59 | 1,55 |
| 5,7 | 2,65 | 1,93 | 2,29 |
| 6,7 | 4,97 | 2,82 | 3,08 |
| 6,7 | 0,31 | 0,31 | 0,32 |
| 7,7 | 0,54 | 0,48 | 0,52 |
| 8,7 | 1,16 | 1,17 | 1,16 |
| 9,7 | 1,92 | 1,92 | 1,89 |
| 11,0 | 3,94 | 4,11 | 4,07 |
| 11,8 | 5,61 | 6,12 | 6,17 |
| 12,7 | 7,76 | 8,29 | 8,35 |
| 13,7 | 9,51 | 10,49 | 10,64 |
| 14,7 | 10,92 | 12,44 | 12,64 |
| 15,7 | 10,88 | 11,34 | 12,39 |
| 16,7 | 10,69 | 12,42 | 12,49 |
| 17,9 | 11,13 | 11,06 | 11,76 |
| 18,9 | 10,99 | 11,31 | 12,39 |
| 19,7 | 10,76 | 11,20 | 12,06 |
| 20,7 | 10,67 | 11,13 | 12,06 |
| 21,7 | 10,74 | 11,24 | 12,10 |
| **max VCD** | 11,13 | 12,44 | 12,64 |

### Experiment 3: Comparison of the cell growths obtained with TC-42 media in which L-valine and L-isoleucine, respectively, are replaced by L-lysyl-L-valine and L-lysyl-L-isoleucine, respectively.

For this experiment a TC-42 media produced by Xell AG, Bielefeld, Germany in which L-lysine, L-valine and L-isoleucine were lacking was used. These amino acids and the dipeptides L-lysyl-L-valine and L-lysyl-L-isoleucine, respectively, were then added to the test media in the concentration listed in table 6.

**Table 6: Concentration of amino acids and dipeptides used in the test media of experiment 3.**

| | | **Reference Media** | | **Lys-Val** | | **Lys-Ile** | |
|---|---|---|---|---|---|---|---|
| | **MW** | **[mM]** | **[mg/l]** | **[mM]** | **[mg/l]** | **[mM]** | **[mg/l]** |
| L-Lysine x HCI | 182,65 | 2,90 | 530,5 | | | | |
| L-Isoleucine | 131,18 | 2,90 | 381,0 | 2,90 | 381,0 | | |
| L-Valine | 117,15 | 2,90 | 340,3 | | | 2,90 | 340,3 |
| L-Lysyl-L-valine | 245,32 | | | 2,90 | 712,5 | | |
| L-Lysyl-L-isoleucine | 259,35 | | | | | 2,90 | 753,27 |

The viable cell densities (VCD) obtained with the media from table 6 at different times are listed in table 7.

**Table 7: Viable cell densities (VCD) and maximum cell densities (max VCD) obtained with the media listed in table 6.**

| **Time** | **Reference** | **Lys-Ile** | **Lys-Val** |
|---|---|---|---|
| 0,0 | 0,44 | 0,44 | 0,43 |
| 2,8 | 2,77 | 1,78 | 2,04 |
| 3,0 | 0,45 | 0,48 | 0,50 |
| 6,8 | 4,35 | 3,36 | 4,37 |
| 6,9 | 0,36 | 0,39 | 0,39 |
| 7,9 | 0,66 | 0,60 | 0,61 |
| 8,8 | 1,21 | 1,04 | 1,15 |
| 9,8 | 2,28 | 1,73 | 2,07 |
| 10,8 | 3,87 | 2,73 | 3,35 |
| 11,8 | 6,38 | 4,62 | 5,79 |
| 12,9 | 8,53 | 5,77 | 8,34 |
| 13,8 | 11,54 | 7,19 | 11,08 |
| 14,8 | 16,25 | 9,27 | 15,45 |
| 15,9 | 16,29 | 9,45 | 16,84 |
| 16,9 | 17,34 | 9,40 | 17,25 |
| 17,9 | 16,73 | 10,56 | 16,27 |
| 18,8 | 14,62 | 10,67 | 14,62 |
| 19,8 | | 9,18 | |
| 21,0 | | 8,77 | |
| **max VCD** | 17,34 | 10,67 | 17,25 |

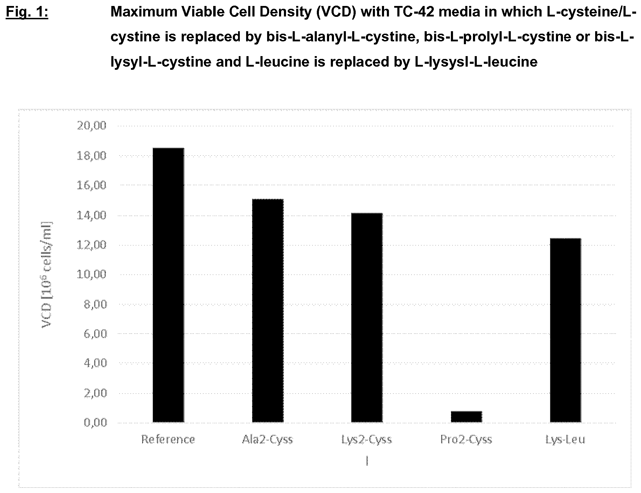

The comparison of a reference medium with other media containing the same molar amount of cysteine and cystine in the form of dipeptides showed that bis-lysyl-L-cysteine led to a performance comparable to bis-alanyl-L-cystine (fig. 1). In both cases, the cells were able to utilize the peptides for growth. Under the same conditions almost no growth can be observed with bis-proly-cystine suggesting that the cells are not able to utilise this dipeptide. This shows that the bis-lysyl-L-cysteine can be used as a replacement for the free amino acids. Compared to bis-alanyl-L-cystine, the bis-lysyl-L-cysteine combines surprisingly high solubility (table 1) with bioavailability.

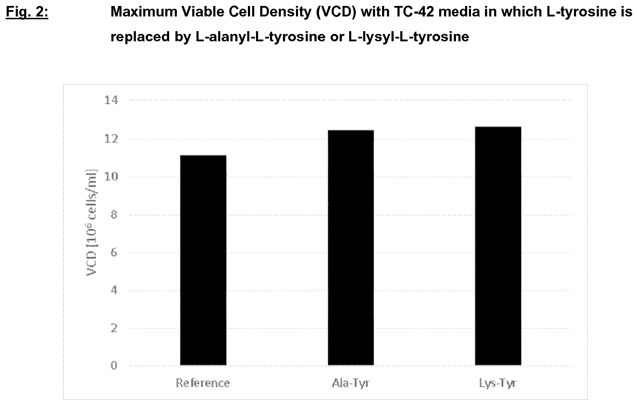

In a second test series media containing the dipeptides L-alanyl-L-tyrosine and L-lysyl-L-tyrosine were compared with the reference medium containing equimolar amounts of tyrosine. As can be seen from fig. 2, the growth curves of the different media show no significant difference. Regarding the maximum cell density both dipeptides even seem to yield slightly higher values. This shows that the L-lysyl-L-tyrosine can be used as a replacement for the free amino acids. Compared to the previously reported L-alanyl-L-tyrosine, the lysyl-L-tyrosine combines surprisingly high solubility (table 1) with bioavailability.

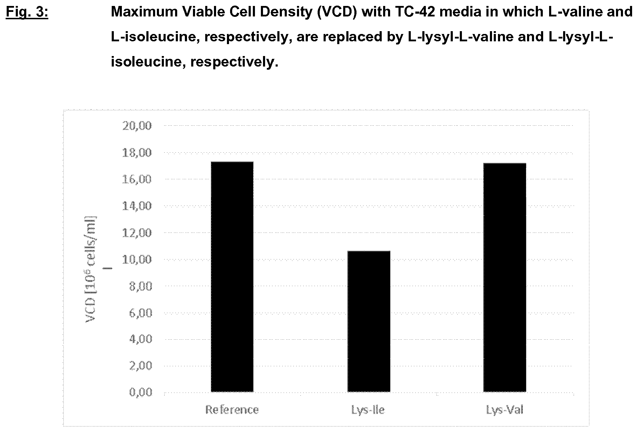

In a further test series media containing L-lysyl-L-valine and L-lysyl- L-isoleucine were compared to a reference medium containing L-lysine or L-valine in equimolar amounts. Whilst L-lysyl-L-valine produced a growth curve and a maximum cell density almost matching those of the reference medium, L-lysyl-L-isoleucine generated a slower growth and a lower maximum cell density (fig. 3). In both cases, the free amino acids could be replaced by the lysyl-peptides.

Comparative tests clearly prove that lysyl dipeptides of cystine and tyrosine are a suitable substitute for the amino acids of low solubility. The exploitation of the lysyl dipeptides is at least as good as that of commercially used L-alanyl dipeptides. The use of lysyl dipeptides does not result in an undesirable increase of an amino acid in the media as the lysine, which is released from the lysyl dipeptides, is present in the usual cell cultures in considerably higher concentrations than alanine.

As the lysyl dipeptides exhibit a considerably higher solubility they can be dissolved much easier and faster than the amino acids or alanyl dipeptides their application seems beneficial in dehydrated culture media that are prepared shortly before use.

Their increased solubility also facilitates the production of concentrations that can be diluted to the desired concentration before use. In particular, the lysyl dipeptides enable higher concentrations and hence higher concentration factors than the free amino acids or the alanyl dipeptides, which have been used to date. Cell cultures on a big scale as used for instance in the production of pharmacologic proteins are often cultured for several weeks. During this period the spent nutrients are frequently replaced. To avoid increasing the volume of the medium as much as possible highly dosed feed media are used. Such feed media may contain nutrients in concentrations by a factor of 5 to 1,000 larger than those of the working media. For this the lysyl dipeptides are particularly suited.

## Claims

1. A culture medium, such as a cell culture medium, comprising at least one dipeptide of 2 amino acids in length, said amino acids being natural amino acids and one of said amino acids being lysine (Lys), wherein the dipeptide does not comprise glycine (Gly) and
glutamic acid (Glu),
wherein the dipeptide is Lys-Xxx,
wherein Xxx is selected from Cys, Cyss, Tyr, Val, Leu, and Ile and
wherein said dipeptide is present in said culture medium at a concentration of from 0.1 to 10 mM.

2. The culture medium of any of the preceding claims, wherein the ratio between lysine and other amino acids is 0.5.

3. The culture medium of any one of the preceding claims, said culture medium further comprising at least one carbohydrate, at least one free amino acid, at least one inorganic salt, a buffering agent and/or at least one vitamin.

4. The culture medium of any one of the preceding claims, wherein said medium does not contain a growth factor.

5. The culture medium of any one of the preceding claims, wherein said culture medium is in liquid form, in form of a gel, a powder, a granulate, a pellet or in form of a tablet.

6. The culture medium of any one of the preceding claims, wherein said cell culture medium is a serum-free medium, or a defined medium.

7. The culture medium of any one of the preceding claims, wherein said culture medium is in 2-fold, 3-fold, 3.33-fold, 4-fold, 5-fold or 10-fold concentrated form, relative to the concentration of said medium in use.

8. Use of a culture medium of any one of claims 1-6 for culturing cells.

9. Use of claim 8, wherein said cells are animal cells or plant cells, preferably mammalian cells.

10. Use of claim 9, wherein said cells are selected from the list consisting of CHO cells, COS cells, VERO cells, BHK cells, HEK cells, HELA cells, AE-1 cells, insect cells, fibroblast cells, muscle cells, nerve cells, stem cells, skin cells, endothelial cells and hybridoma cells.

11. Use of a dipeptide of 2 amino acids in length, said amino acids being natural amino acids, and one amino acid being Lys, and said dipeptide not comprising Gly, Glu, Asn, Ala and
Gln, wherein the dipeptide is Lys-Xxx,
wherein Xxx is selected from Cys, Cyss, Tyr, Val, Leu, and Ile and
wherein said dipeptide is present in said culture medium at a concentration of from 0.1 to 10 mM, in a culture medium for culturing cells.

12. Method of manufacturing a cell culture product comprising the steps of
providing a cell capable of producing said cell culture product;
contacting said cell with a culture medium of any one of claims 1-6; and
obtaining said cell culture product from said culture medium or from said cell.

13. Method of claim 12, wherein said cell culture product is selected from the group consisting of therapeutic protein, diagnostic protein, polysaccharide, such as heparin, antibody, monoclonal antibody, growth factor, interleukin, virus, virus-like particle and enzyme.

## Patentansprüche

1. Kulturmedium, beispielsweise Zellkulturmedium, umfassend wenigstens ein Dipeptid mit einer Länge von 2 Aminosäuren, wobei es sich bei den Aminosäuren um natürliche Aminosäuren und bei einer der Aminosäuren um Lysin (Lys) handelt, wobei das Dipeptid nicht Glycin (Gly) und Glutaminsäure (Glu) umfasst, wobei es sich bei dem Dipeptid um Lys-Xxx handelt, wobei Xxx ausgewählt ist aus Cys, Cyss, Tyr, Val, Leu und Ile, und wobei das Dipeptid im Kulturmedium in einer Konzentration von 0,1 bis 10 mM vorliegt.

2. Kulturmedium nach einem der vorhergehenden Ansprüche, wobei das Verhältnis zwischen Lysin und anderen Aminosäuren 0,5 beträgt.

3. Kulturmedium nach einem der vorhergehenden Ansprüche, wobei das Kulturmedium ferner wenigstens ein Kohlenhydrat, wenigstens eine freie Aminosäure, wenigstens ein anorganisches Salz, ein Pufferungsmittel und/oder wenigstens ein Vitamin umfasst.

4. Kulturmedium nach einem der vorhergehenden Ansprüche, wobei das Medium keinen Wachstumsfaktor enthält.

5. Kulturmedium nach einem der vorhergehenden Ansprüche, wobei das Kulturmedium in flüssiger Form, in Form eines Gels, als Pulver, als Granulat, als Pellet oder in Form einer Tablette vorliegt.

6. Kulturmedium nach einem der vorhergehenden Ansprüche, wobei es sich bei dem Zellkulturmedium um ein serumfreies Medium oder ein definiertes Medium handelt.

7. Kulturmedium nach einem der vorhergehenden Ansprüche, wobei das Kulturmedium in 2-fach, 3-fach, 3,33-fach, 4-fach, 5-fach oder 10-fach konzentrierter Form bezüglich der Konzentration des Mediums bei Verwendung vorliegt.

8. Verwendung eines Kulturmediums nach einem der Ansprüche 1-6 zur Kultivierung von Zellen.

9. Verwendung nach Anspruch 8, wobei es sich bei den Zellen um Tierzellen oder Pflanzenzellen, bevorzugt um Säugerzellen handelt.

10. Verwendung nach Anspruch 9, wobei die Zellen ausgewählt sind aus der Liste bestehend aus CHO-Zellen, COS-Zellen, VERO-Zellen, BHK-Zellen, HEK-Zellen, HELA-Zellen, AE-1-Zellen, Insektenzellen, Fibroblasten-Zellen, Muskelzellen, Nervenzellen, Stammzellen, Hautzellen, Endothelzellen und Hybridomzellen.

11. Verwendung eines Dipeptids mit einer Länge von 2 Aminosäuren, wobei es sich bei den Aminosäuren um natürliche Aminosäuren und bei einer Aminosäure um Lys handelt und das Dipeptid nicht Gly, Glu, Asn, Ala und Gln umfasst, wobei es sich bei dem Dipeptid um Lys-Xxx handelt, wobei Xxx ausgewählt ist aus Cys, Cyss, Tyr, Val, Leu und Ile, und wobei das Dipeptid im Kulturmedium in einer Konzentration von 0,1 bis 10 mM vorliegt, in einem Kulturmedium zur Kultivierung von Zellen.

12. Verfahren zur Erzeugung eines Zellkulturprodukts, umfassend die Schritte des
Bereitstellens einer Zelle mit der Fähigkeit zur Produktion des Zellkulturprodukts;
In-Kontakt-Bringens der Zelle mit einem Kulturmedium nach einem der Ansprüche 1-6; und
Gewinnens des Zellkulturprodukts aus dem Kulturmedium oder aus der Zelle.

13. Verfahren nach Anspruch 12, wobei das Zellkulturprodukt ausgewählt ist aus der Gruppe bestehend aus therapeutischem Protein, diagnostischem Protein, Polysaccharid, beispielsweise Heparin, Antikörper, monoklonaler Antikörper, Wachstumsfaktor, Interleukin, Virus, virusähnliches Partikel und Enzym.

## Revendications

1. Milieu de culture, tel qu'un milieu de culture cellulaire, comprenant au moins un dipeptide de 2 acides aminés de longueur, lesdits acides aminés étant des acides aminés naturels et l'un desdits acides aminés étant la lysine (Lys), dans lequel le dipeptide ne comprend pas de glycine (Gly) et d'acide glutamique (Glu),
dans lequel le dipeptide est Lys-Xxx,
dans lequel Xxx est choisi parmi Cys, Cyss, Tyr, Val, Leu et Ile, et
dans lequel ledit dipeptide est présent dans le milieu de culture à une concentration de 0,1 à 10 mM.

2. Milieu de culture selon l'une quelconque des revendications précédentes, dans lequel le rapport entre la lysine et les autres acides aminés est de 0,5.

3. Milieu de culture selon l'une quelconque des revendications précédentes, ledit milieu de culture comprenant en outre au moins un glucide, au moins un acide aminé libre, au moins un sel inorganique, un agent tampon et/ou au moins une vitamine.

4. Milieu de culture selon l'une quelconque des revendications précédentes, ledit milieu ne contenant pas de facteur de croissance.

5. Milieu de culture selon l'une quelconque des revendications précédentes, ledit milieu de culture se présentant sous forme liquide, sous forme d'un gel, d'une poudre, d'un granulé, d'une pastille ou sous forme d'un comprimé.

6. Milieu de culture selon l'une quelconque des revendications précédentes, ledit milieu de culture étant un milieu sans sérum, ou un milieu défini.

7. Milieu de culture selon l'une quelconque des revendications précédentes, ledit milieu de culture se présentant sous une forme concentrée 2x, 3x, 3,33x, 4x, 5x ou 10x, par rapport à la concentration dudit milieu en cours d'utilisation.

8. Utilisation d'un milieu de culture selon l'une quelconque des revendications 1 à 6 pour la culture de cellules.

9. Utilisation selon la revendication 8, lesdites cellules étant des cellules animales ou des cellules végétales, de préférence des cellules de mammifère.

10. Utilisation selon la revendication 9, lesdites cellules étant choisies dans la liste consistant en les cellules CHO, les cellules COS, les cellules VERO, les cellules BHK, les cellules HEK, les cellules HELA, les cellules AE-1, les cellules d'insectes, les cellules fibroblastes, les cellules musculaires, les cellules nerveuses, les cellules souches, les cellules cutanées, les cellules endothéliales et les cellules d'hybridome.

11. Utilisation d'un dipeptide de 2 acides aminés de longueur, lesdits acides aminés étant des acides aminés naturels, et un acide aminé étant Lys, et ledit peptide ne comprenant pas Gly, Glu, Asn, Ala et Gln, le dipeptide étant Lys-Xxx,
Xxx étant choisi parmi Cys, Cyss, Tyr, Val, Leu et Ile, et ledit dipeptide étant présent dans ledit milieu de culture à une concentration de 0,1 à 10 mM, dans un milieu de culture pour la culture de cellules.

12. Procédé de fabrication d'un produit de culture cellulaire comprenant les étapes de
fourniture d'une cellule capable de produire ledit produit de culture cellulaire ;
mise en contact de ladite cellule avec un milieu de culture selon l'une quelconque des revendications 1 à 6 ; et
obtention dudit produit de culture cellulaire à partir dudit milieu de culture ou de ladite cellule.

13. Procédé selon la revendication 12, dans lequel ledit produit de culture cellulaire est choisi dans le groupe consistant en une protéine thérapeutique, une protéine diagnostique, un polysaccharide tel que l'héparine, un anticorps, un anticorps monoclonal, un facteur de croissance, une interleukine, un virus, une particule pseudo-virale et une enzyme.
